# EUROPEAN PATENT APPLICATION

(11) **EP 2 322 532 A1**
(43) Date of publication of application: **18.05.2011**
(21) Application number: 09806725.9
(22) Date of filing: 11.08.2009
(51) Int. Cl.: C07H 5/06, C07H 1/06, C07H 7/027, G01N 27/62

(54) **METHOD FOR RELEASING REDUCING GLYCAN BY AMMONIUM SALT**

(30) Priority: 12.08.2008 JP 2008208274
(71) Applicant: National University Corporation Hokkaido University, Sapporo-shi Hokkaido 060-0808 (JP); Shionogi&Co., Ltd., Osaka-shi, Osaka 5410045 (JP)
(72) Inventor: The designation of the inventor has not yet been filed
(74) Representative: UEXKÜLL & STOLBERG
(86) International application number: PCT/JP2009/064203
(87) International publication number: WO 2010/018834

(57) **Abstract**

An objective of the present invention is to provide a glycan releasing method which can be applied to construction of a system for automation of glycan analysis, and particularly a glycan releasing method capable of analyzing an O-linked glycan.
The objective could be achieved as a result of finding that the pH is lowered by using an ammonium salt or ammonium ion in the absence of concentrated aqueous ammonia, not using concentrated aqueous ammonia, thus drastically avoiding an undesired side reaction such as a peeling reaction or the like. Therefore, the present invention provides a method of producing an O-linked glycan from a glycan-binding substance having the O-linked glycan, the method includes the steps of (A) bringing an ammonium salt or ammonium ion into contact with the glycan-binding substance in the absence of concentrated aqueous ammonia (for example, under the conditions of the pH of about 7 or higher and lower than about 11); (B) neutralizing the reaction solution obtained in step (A); and (C) collecting the released glycan.

## Description

### TECHNICAL FIELD

The present invention relates to a method of releasing a reducing glycan from glycoconjugates using a basic compound. The present invention can be utilized in a biochemical and clinical study, and can be applied to glycoprotein, glycopeptide, O-linked glycan glycoside, oligosaccharide and the like.

### BACKGROUND ART

Analysis of a glycan in biological molecules has been performed for various purposes, and its importance has suddenly increased recently.

Heretofore, an attempt for analyzing an O-linked glycan in biologically relevant molecules exists. For example, an alkaline β-elimination method and an anhydrous hydrazine decomposition method are methods those are often used. However, since a strong reducing agent is used for suppressing a peeling reaction of a side reaction in the alkaline β-elimination method, the reducing terminal of the released glycan is converted into an alditol. Therefore, restriction on the subsequent handling of the sample arises. Also, it is difficult to carry out the reaction on a small scale since a large amount of biologically relevant molecules are required. The anhydrous hydrazine decomposition method enables release of a glycan while maintaining the reducing terminal, but has a drawback in that the operation is complicated and an N-acetyl group and an N-glycolyl group on the glycan are removed. In addition, since the reagent itself is a dangerous material (poisonous), close attention must be paid when used.

There is also a glycan releasing method using concentrated aqueous ammonia solution (Patent Document 1). The above problems have generally been solved by this method. However, since concentrated aqueous ammonia is used, it is difficult to operate and handle, and also to construct a system for automation of glycan analysis.

Thus, it is desirable to develop a glycan releasing method which can be applied to the construction of a system for automation of glycan analysis, and particularly a glycan releasing method capable of analyzing an O-linked glycan.
Patent Document 1: Specification of U.S. Patent Application Publication No. 2004-0096948

### DISCLOSURE OF THE INVENTION

### PROBLEMS TO BE SOLVED BY THE INVENTION

An objective of the present invention is to provide a glycan releasing method which can be applied to construction of a system for automation of glycan analysis, and particularly a glycan releasing method capable of analyzing an O-linked glycan.

### MEANS FOR SOLVING THE PROBLEMS

The above objective has been achieved by finding that an undesired side reaction such as a peeling reaction can be drastically avoided by using an ammonium salt or ammonium ion in the absence of concentrated aqueous ammonia, and not using concentrated aqueous ammonia.

Therefore, the present invention relates to a method of releasing a reducing glycan from glycoconjugates using a basic compound. Portions of subject matters included in the above glycoconjugates are biological molecules, glycoprotein, glycolipid, glycosaminoglycan and glycopeptide. In addition, O-linked glycan glycoside is included. According to the present invention, it is possible to release a reducing glycan from a glycoconjugate with satisfactory reproducibility by using a substance capable of generating an ammonium salt or ammonium ion, which enables simple and easy handling in comparison to the alkaline β-elimination method, the anhydrous hydrazine decomposition method and the O-linked glycan releasing method using concentrated aqueous ammonia solution as previously reported, for example, an ammonium salt powder or the like while suppressing a peeling reaction as an undesired side reaction as low as possible. The released glycan can be labeled with a fluorescent dye at the reducing terminal thereof, or can be subjected to purification and collection by reacting a glycan reducing terminal with an aminooxy compound or a hydrazide compound, which is called as a "glycoblotting method". Thereafter,qualitative and quantitative analysis can be performed by HPLC or mass spectrometry.

In one aspect, the present invention provides a method of producing an O-linked glycan from a glycan-binding substance containing the O-linked glycan, the method includes the steps of:
(A) bringing an ammonium salt or ammonium ion into contact with the glycan-binding substance in the absence of concentrated aqueous ammonia;
(B) neutralizing or acidifying the reaction solution obtained in step (A) (acidifying the reaction solution after neutralization in some cases); and
(C) collecting the released glycan.

Alternatively, in another aspect, the present invention provides a method of producing an O-linked glycan from a glycan-binding substance containing the O-linked glycan, the method includes the steps of:
(A) adding an ammonium salt to the glycan-binding substance in the absence of concentrated aqueous ammonia (adding an aqueous ammonium salt solution in some cases);
(B) neutralizing or acidifying the reaction solution obtained in step (A) (acidifying the reaction solution after neutralization in some cases); and
(C) collecting the released glycan.

In another aspect, the present invention provides a method of producing an O-linked glycan from a glycan-binding substance containing the O-linked glycan, the method includes the steps of:
(A) bringing an ammonium salt or ammonium ion into contact with the glycan-linked substance under the condition in which the pH is about 7 or higher and lower than about 11;
(B)neutralizing or acidifying the reaction solution obtained in step (A) (acidifying the reaction solution after neutralization in some cases); and
(C) collecting the released glycan.

Alternatively, in another aspect, the present invention provides a method of producing an O-linked glycan from a glycan-binding substance containing the O-linked glycan, the method includes the steps of:
(A) adding an ammonium salt to the glycan-binding substance under the condition in which the pH is about 7 or higher and lower than about 11 (adding an aqueous ammonium salt solution in some cases);
(B) neutralizing or acidifying the reaction solution obtained in step (A) (acidifying the reaction solution after neutralization in some cases); and
(C) collecting the released glycan.

In still another aspect, the present invention provides a method of detecting an O-linked glycan in a sample, the method includes the steps of:
(A) bringing an ammonium salt or ammonium ion into contact with the sample in the absence of concentrated aqueous ammonia;
(B) neutralizing or acidifying the reaction solution obtained in step (A) (acidifying the reaction solution after neutralization in some cases); and
(C) analyzing the released glycan.

Alternatively, in another aspect, the present invention provides a method of detecting an O-linked glycan in a sample, the method includes the steps of:
(A) adding an ammonium salt to the sample in the absence of concentrated aqueous ammonia (adding an aqueous ammonium salt solution in some cases);
(B) neutralizing or acidifying the reaction solution obtained in step (A) (acidifying the reaction solution after neutralization in some cases); and
(C) analyzing the released glycan.

In still another aspect, the present invention provides a method of detecting an O-linked glycan in a sample, the method includes the steps of:
(A) bringing an ammonium salt or ammonium ion into contact with the sample under the condition in which the pH is about 7 or higher and lower than about 11;
(B) neutralizing or acidifying the reaction solution obtained in step (A) (acidifying the reaction solution after neutralization in some cases); and
(C) analyzing the released glycan.

Alternatively, in another aspect, the present invention provides a method of detecting an O-linked glycan in a sample, the method includes the steps of:
(A) adding an ammonium salt to the sample under the condition in which the pH is about 7 or higher and lower than about 11 (adding an aqueous ammonium salt solution in some cases) ;
(B) neutralizing or acidifying the reaction solution obtained in step (A) (acidifying the reaction solution after neutralization in some cases); and
(C) analyzing the released glycan.

In the detection method of the present invention, when a reducing glycan is analyzed, it is preferred that the solution is acidified (for example, the pH of about 3 to about 5).

In one embodiment, in the method of the present invention, the contact with the ammonium salt or ammonium ion, or the addition of the ammonium salt is achieved by the condition in which the ammonium salt is added in an amount ranging from an amount corresponding to half of the saturated concentration to an amount corresponding to the saturated concentration or more.

In another embodiment, in the method of the present invention, the contact with the ammonium salt or ammonium ion, or the addition of the ammonium salt is achieved by the condition in which the ammonium salt is added in an amount corresponding to the saturated concentration or more.

In still another embodiment, in the method of the present invention, the contact with the ammonium salt or ammonium ion, or the addition of the ammonium salt is achieved by adding a substance capable of generating the ammonium salt or ammonium ion to a solution of the glycan-binding substance in the form of a powder.

In still another embodiment, the method of the present invention further includes the step of heating after the contact with the ammonium salt or ammonium ion, or the addition of the ammonium salt.

In still another embodiment, heating in the method of the present invention is performed at 50°C to 80°C for 10 to 100 hours.

In still another embodiment, in the method of the present invention, the pH of the ammonium salt or ammonium ion is about 8.3 or higher and about 10.8 or lower. Preferably, the pH can be about 8.5 or higher and about 10.5 or lower, about 8.5 or higher and about 10 or lower, about 9 or higher and about 10 or lower, or about 9.5 or higher and about 9.9 or lower.

In still another embodiment, the ammonium salt used in the method of the present invention contains at least one salt selected from the group consisting of ammonium carbonate, ammonium bicarbonate and ammonium carbamate.

In still another embodiment, neutralization in the method of the present invention is performed using an acid or ion exchange resin.

In still another embodiment, the glycan-binding substance or sample used in the present invention is contained in serum, a cultured cell extract or a tissue sample.

In still another embodiment, the glycan-binding substance or sample used in the present invention is contained in urea, plasma and the like.

In still another embodiment, the acidic substance used in the step of neutralizing or acidifying in step (B) in the method of the present invention is not particularly limited, and is preferably an acidic substance which neutralizes the solution and forms a volatile salt, and the step is performed using acetic acid, trifluoroacetic acid, formic acid and the like. In the same step, "acidifying" includes adjusting the solution to a "pH of about 3 to about 5".

In still another embodiment, collection of a glycan in the method of the present invention is achieved by a glycoblotting method.

In still another embodiment, collection of a glycan in the method of the present invention can also be achieved by labeling (for example, fluorescence label, etc.) the released O-linked glycan. In this case, analysis can be performed by HPLC or mass spectrometry.

In still another embodiment, the glycan-binding substance in the method of the present invention is a biological molecule such as glycoprotein, glycopeptide, proteoglycan, glycosaminoglycan, glycolipid, carbohydrate-nucleic acid complex or glycopeptidolipid (GPL).

In still another embodiment, the glycan-binding substance having an O-linked glycan as the subject matter in the method of the present invention is a substance containing serine or threonine.

In a preferred embodiment, the present invention provides a method of producing an O-linked glycan from a glycan-binding substance containing the O-linked glycan, the method includes the steps of:
(A) adding an ammonium bicarbonate, ammonium carbonate or ammonium carbamate powder or an aqueous solution of an ammonium bicarbonate, ammonium carbonate or ammonium carbamate powder to an aqueous solution containing the glycan-binding substance so that the aqueous solution satisfies the half saturation to saturation condition, followed by heating;
(B) neutralizing the reaction solution obtained in step (A) with an acid (for example, acetic acid, formic acid, trifluoroacetic acid, etc.), (followedby further acidifying the reaction solution so that the pH is adjusted to about 3 to about 5 and converting the produced glycosylamine glycan into a reducing released glycan, if necessary); and
(C) collecting the released glycan using a glycan capturing carrier.

In another preferred embodiment, the present invention provides a method of detecting an O-linked glycan, the method includes the steps of:
(A) adding an ammonium bicarbonate, ammonium carbonate or ammonium carbamate powder or an aqueous solution of an ammonium bicarbonate, ammonium carbonate or ammonium carbamate powder to an aqueous solution containing the glycan-binding substance so that the aqueous solution satisfies the half saturation to saturation condition, followed by heating;
(B) acidifying the reaction solution obtained in step (A) (adjusting the pH of the reaction solution to about 3 to about 5, preferably); and
(C) collecting the released glycan using a glycan capturing carrier, and analyzing the released glycan by mass spectrometry.

In a still preferred embodiment, the present invention provides a method of producing an O-linked glycan from a glycan-binding substance containing the O-linked glycan, the method includes the steps of:
(A) adding an ammonium carbonate or ammonium carbamate powder or an aqueous solution of an ammonium bicarbonate, ammonium carbonate or ammonium carbamate powder to an aqueous solution containing the glycan-binding substance so that the aqueous solution satisfies the half saturation to saturation condition, followed by heating at about 60°C;
(B') acidifying the reaction solution obtained in step (A) with acetic acid, formic acid, trifluoroacetic acid and the like (for example, adjusting to the pH of about 3 to about 5); and
(C) collecting the released glycan using a glycan capturing carrier (a resin having a hydrazide group, such as BlotGlyco Series (available from Sumitomo Bakelite Co., Ltd.) or AffiGel Hz Series (available from Bio-Rad Laboratories, Inc.)).

In still another preferred embodiment, the present invention provides a method of detecting an O-linked glycan, the method includes the steps of:
(A) adding an ammonium carbonate or ammonium carbamate powder or an aqueous solution of an ammonium bicarbonate, ammonium carbonate or ammonium carbamate powder to an aqueous solution containing the glycan-binding substance so that the aqueous solution satisfies the half saturation to saturation condition, followed by heating at about 60°C;
(B) acidifying the reaction solution obtained in step (A) with acetic acid, formic acid, trifluoroacetic acid and the like (for example, adjusting to the pH of about 3 to about 5); and
(C) collecting the released glycan using a glycan capturing carrier (BlotGlyco Series (available from Sumitomo Bakelite Co., Ltd.) or AffiGel Hz Series (available from Bio-Rad Laboratories, Inc.), and analyzing the released glycan by mass spectrometry.

In another aspect, the present invention provides a method of detecting an O-linked glycan in a sample which is expected to contain a glycan-binding substance having the O-linked glycan, the method includes the steps of:
(A) bringing an ammonium salt or ammonium ion into contact with the glycan-binding substance;
(B) acidifying the reaction solution obtained in step (A) (preferably, adjusting to the pH of about 3 to about 5); and
(C) analyzing the released glycan.

Alternatively, in another aspect, the present invention provides a method of detecting an O-linked glycan in a sample which is expected to contain a glycan-binding substance having the O-linked glycan, the method includes the steps of:
(A) adding an ammonium salt to the glycan-binding substance;
(B) acidifying the reaction solution obtained in step (A) (preferably, adjusting to the pH of about 3 to about 5); and
(C) analyzing the released glycan.

Alternatively, in another aspect, the present invention provides a method of detecting an O-linked glycan in a sample which is expected to contain a glycan-binding substance having the O-linked glycan, the method includes the steps of:
(A) adding an ammonium salt to the glycan-binding substance;
(B) acidifying the reaction solution obtained in step (A) (preferably, adjusting to the pH of about 3 to about 5); and
(C) collecting the released glycan using a glycan capturing carrier (BlotGlyco Series (available from Sumitomo Bakelite Co., Ltd.) or AffiGel Hz series (available from Bio-Rad Laboratories, Inc.), and analyzing the released glycan by mass spectrometry.

In one embodiment, where analysis is performed in the method of the present invention, the analysis is carried out by mass spectrometry, preferably MALDI-TOF-MS or LC-ESI-MS, and more preferably MALDI-TOF-MS.

In another aspect, the present invention provides a composition for producing or separating an O-linked glycan from a glycan-binding substance having the O-linked glycan, the composition containing an ammonium salt or ammonium ion.

In one embodiment, the ammonium salt or ammonium ion used in the composition of the present invention contains at least one salt selected from the group consisting of ammonium carbonate, ammonium bicarbonate and ammonium carbamate or an ion derived therefrom.

In another embodiment, the ammonium salt or ammonium ion used in the composition of the present invention contains ammonium bicarbonate or ammonium carbamate.

In another embodiment, the glycan-binding substance is contained in serum, a cultured cell extract or a tissue sample.

In another aspect, the present invention provides an ammonium salt or ammonium ion for producing or separating an O-linked glycan from a glycan-binding substance having the O-linked glycan.

In the other aspect, the present invention provides an ammonium salt or ammonium ion for detecting an O-linked glycan in a sample.

In the other aspect, the present invention provides use of an ammonium salt or ammonium ion for producing or separating an O-linked glycan from a glycan-binding substance having the O-linked glycan.

In the other aspect, the present invention provides use of an ammonium salt or ammonium ion for producing a drug for producing or separating an O-linked glycan from a glycan-binding substance having the O-linked glycan.

In the other aspect, the present invention provides use of an ammonium salt or ammonium ion for detecting an O-linked glycan in a sample.

In the other aspect, the present invention provides use of an ammonium salt or ammonium ion for producing a drug for detecting an O-linked glycan in a sample.

In another aspect, the present invention provides a kit for producing or separating an O-linked glycan from a glycan-binding substance having the O-linked glycan, the kit includes:
(A) an ammonium salt or ammonium ion,
(B) means for neutralizing (and/or means for acidifying) the ammonium salt or ammonium ion, and
(C) means for collecting a glycan.

In another aspect, the present invention provides a system used for detecting an O-linked glycan in a sample which is expected to contain a glycan-binding substance having the O-linked glycan, the system includes:
(A) an ammonium salt or ammonium ion,
(B) means for neutralizing (and/or means for acidifying) the ammonium salt or ammonium ion, and
(C) means for detecting a glycan.

In still another aspect, the present invention provides a method of analyzing a disease in which a variation in an O-linked glycan is observed, specifying an etiology and making a diagnosis, utilizing the method of detecting an O-linked glycan of the present invention.

In still another aspect, the present invention is capable of screening subject matter depending on an O-linked glycan utilizing the method of detecting an O-linked glycan of the present invention. Examples of subj ect matter to be screened include, but are not limited to, a living body itself (for example, pathogenic bacteria, etc.), a biological sample, a medicament or a candidate thereof.

### EFFECT OF THE INVENTION

According to the present invention, various operational problems due to the conventional art are solved and a reducing free oligosaccharide is prepared simply and easily. Particularly, it is possible to determine a glycan structure in biologically relevant molecules quantitatively and highly reproducibly by using a combination of glycoblotting (including sialic acid modification) and mass spectrometry. When performing the glycoblotting method, it is also possible to quench acidic charge through methyl esterification of sialic acid so as to enable quantitative mass spectrometry.

In a preferred embodiment, ammonia carbonate or an ammonium carbamate powder having the saturated concentration or more (15 to 20 mg/20 µl) is added to an aqueous solution containing biologically relevant molecules having an O-linked glycan, and then the mixture is subjected to a heat treatment at 60°C for 20 hours to 40 hours. The reaction solution is neutralized with an acid such as acetic acid, formic acid or trifluoroacetic acid (preferably, an acid capable of producing a volatile salt) or an ion exchange resin (further acidified) to obtain an aqueous, and then the solution can optionally be freeze-dried. The released glycan is subjected to glycoblotting to be purified and collected, and can be qualitatively and quantitatively analyzed by mass spectrometry.

Thus, according to the present invention, it becomes possible to perform the operation, which is complicated and difficult for a conventional O-linked glycan releasing method from biologically relevant molecules, quickly and easily. It is also possible to release an O-linked glycan from a small amount of biologically relevant molecules. It becomes possible to minimize the peeling reaction by optimization of the reaction conditions, and thus quantitative analysis with high reproducibility can be performed. Therefore, it becomes possible to automate O-linked glycan analysis for the first time.

### BRIEF DESCRIPTION OF THE DRAWINGS

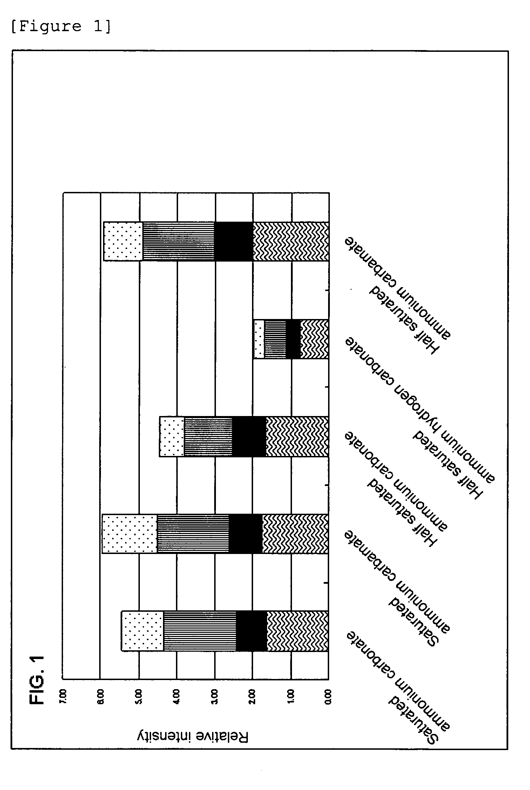

Fig. 1 illustrates the released amount of various O-linked glycans after treatment with various ammonium salts performed in Example 1 by a relative intensity. 1.00 is an internal standard. The treatment condition is 60°C for 20 hours, and BSM = 800 µg: internal standard = GN4 (2, 500 pmol) . Polka dots (top) denote (HexNAc)2(NeuAc)1. Vertical stripes (second from the top) denote (HexNAc)2(NeuGc)1. Black (third from the top) denotes (HexNAc) 1 (NeuAc) 1. Wavy lines (bottom) denote (HexNAc) 1 (NeuGc) 1. NeuAc represents N-acetylneuraminic acid, NeuGc represents N-glycolylneuraminic acid. HexNAc denotes N-acetylhexosamine.

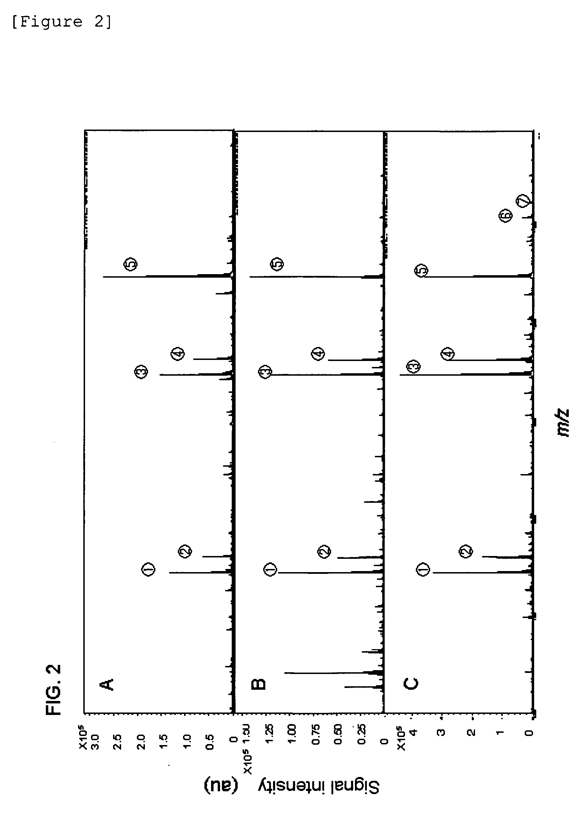

Fig. 2 illustrates a mass spectrum of BSM performed in Example 1. The conditions of dissociation of various glycan bonding are as follows. Hydrazine decomposition: 60°C for 6 hours <denoted by A>, (NH₄)₂CO₃: 60°C for 20 hours <denoted by B>, and NH₂COONH₄: 60°C for 20 hours <denoted by C>. Here, the results are obtained by adding 2, 500 pmol of chitotetraose, as each internal standard, relative to 400 µg of BSM and exchanging with pentafluoro-benzylhydroxylamine [BOA(F)] or benzylhydroxylamine (BOA) are shown. The ordinate denotes the relative intensity (a.u.), where as, the absciss as denotes the mass (m/z).

In the graph, circled numbers denote the followings. The circled number 1 denotes (HexNAc) 1 (NeuAc) 1. 2 denotes (HexNAc)1(NeuGc)1. 3 denotes (HexNAc)2(NeuAc)1. 4 denotes (HexNAc) 2 (NeuGc) 1. 5 denotes (HexNAc) 4. 6 denotes (HexNAc)2(Hex)1(NeuAc)1. 7 denotes (HexNAc)2(Hex)1(deoxyHex)1(NeuAc)1. Here, Hex denotes hexose and deoxyHex denotes deoxyhexose.

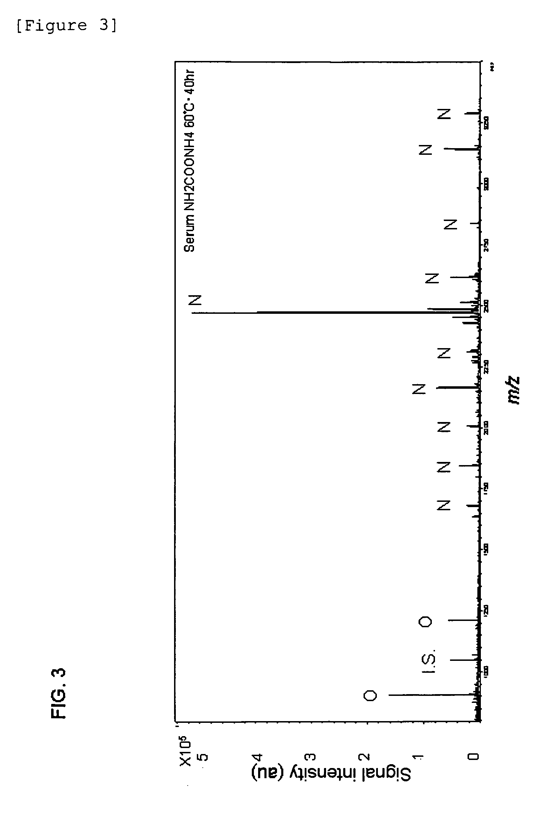

Fig. 3 illustrates that, glycoprotein glycan can be released by treating human serum with saturated ammonium carbamate, shown by both analytical results (mass spectrum) of mass spectrometry of an O-linked glycan and an N-linked glycan. The case of using ammonium carbamate NH₂COONH₄ was examined. The heating condition used was 60°C for 40 hours. The ordinate denotes the relative intensity (a.u.), whereas, the abscissas denotes the mass (m/z). Symbols in the graph are as follows. I.S.: internal standard; O: O-linked glycan; N: N-linked glycan.

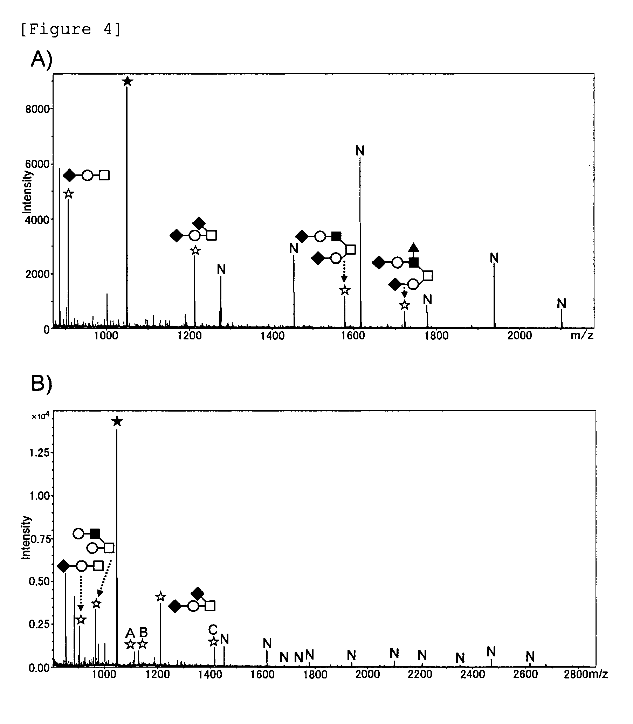

Fig. 4 illustrates analysis by MALDI-TOF mass spectrometry of a glycan in a biological sample obtained by a glycan releasing method using an ammonium carbamate powder. A: Human breast cancer cell line MCF-7. B: Rat kidney formalin-fixed paraffin-embedded sample. After extracting a protein fraction, the released glycan was purified and collected by a glycoblotting method using BlotGlyco beads and a mass spectrum was obtained. Asterisk represents an O-glycan. Label N in the figure is a peak derived from an N-glycan. The symbol ◆ (black diamond) denotes NeuAc (sialic acid), o (white circle) denotes Hex (hexose), □ (white square) denotes HexNAc (GalNAc), ■ (black square) denotes HexNAc (GlcNAc), and Δ (white triangle) denotes dHex (fucose). Black asterisk existing at about 1, 000 denotes the internal standard, and other white asterisks denote an O-linked glycan. The symbols A, B and C on the white asterisk denotes an O-linked glycan in which the composition is further defined. A is Hex2HexNAc2dHex1 (i.e. two hexoses, two N-acetylhexoses, one deoxyhexose), B is Hex3HexNAc2 (three hexoses, two N-acetylhexoses), and C is Hex1HexNAc2NeuAc2 (one hexose, two N-acetylhexoses, two sialic acids).

### MODE FOR CARRYING OUT THE INVENTION

The present invention will be described below. It should be understood that an expression of a singular form includes the concept of its plural form over the entire present description unless otherwise specified. It should also be understood that terms as used herein mean those used commonly in the relevant technical field unless otherwise specified.

### (Terms)

Definitions of terms used particularly in the present description are listed below.

As used herein, a "glycan" refers to a compound obtained by connecting one or more sugar units (a monosaccharide and/or a derivative of a monosaccharide). In case two or more sugar units are connected, the respective sugar units are combined by dehydration condensation forming a glycosidic bond. Examples of such a glycan include, but are not limited to, various glycans, for example, glycoconjugates (constituted of glucose, galactose, mannose, fucose, xylose, N-acetylglucosamine, N-acetylgalactosamine, sialic acid, uronic acid, a complex and a derivative thereof) contained in the living body, decomposed polysaccharides, glycans decomposed or derived from complex biological molecules such as glycoprotein, glycopeptide, proteoglycan, glycosaminoglycan and glycolipid. Therefore, in the present description, the glycan can be used interchangeably with "polysaccharide", "glycoside", "carbohydrate" and "oligosaccharide".

As used herein, a "monosaccharide" refers to a compound which is not hydrolyzed into simpler molecules and is represented by the general formula: CₙH₂ₙOₙ. Here, those in which n = 2, 3, 4, 5, 6, 7, 8, 9 and 10 are respectively referred to as diose, triose, tetrose, pentose, hexose, heptose, octose, nonose and decose. In general, the monosaccharide corresponds to an aldehyde or a ketone of a chain polyhydric alcohol, and the former is called an aldose, while the latter is called a ketose.

As used herein, a "derivative of a monosaccharide" refers to one in which one or more hydroxyl groups on the monosaccharide are substituted with other substituent(s) and the obtained substance is not within the scope of the monosaccharide. Examples of such a derivative of a monosaccharide include, but are not limited to, a saccharide having a carboxyl group (for example, aldonic acid (for example, D-gluconic acid obtained by oxidation of D-glucose) in which oxidation is performed at the C-1 position to form a carboxylic acid), uronic acid (D-glucuronic acid obtained by oxidation of D-glucose) in which a terminal C atom is converted into carboxylic acid, a saccharide (for example, N-acetyl-D-glucosamine or N-acetyl-D-galactosamine) having an amino group or a derivative of an amino group (for example, an acetylated amino group), a saccharide having both an amino group and a carboxyl group (for example, N-acetylneuraminic acid (sialic acid) or N-acetylmuramic acid), a deoxylated saccharide (for example, 2-deoxy-D-ribose), sulfated saccharide having a sulfate group, and a phosphorylated saccharide having a phosphate group. Alternatively, in a saccharide having a hemiacetal structure formed, which is a glycoside having an acetal structure reacted with an alcohol is also within the scope of the derivative of a monosaccharide.

As used herein, a "glycan-binding substance" refers to a substance in which a glycan is bound to a substance other than the glycan (for example, protein, lipid, etc.). Such a glycan-binding substance is often found in the living body and examples thereof include, but are not limited to, various glycan-binding substances, for example, complex biological molecules such as glycoprotein, glycopeptide, proteoglycan, glycosaminoglycan and glycolipid, and glycans decomposed or derived therefrom. In addition, the glycan-binding substance may be one which is contained in any sample. For example, it is possible to use, as the glycan-binding substance, those contained in a body fluid such as serum, a cultured cell extract or a tissue sample (for example, a formalin-fixed paraffin-embedded (FFPE) tissue sample). It has recently become apparent that a function to be exerted by the glycan-binding substance largely varies depending on the kind and amount of the glycan bound, and importance of analysis thereof is increasing.

In the present invention, examples of the term "glycoprotein" include, but are not limited to, enzymes, hormones, cytokines, antibodies, vaccines, receptors, and serum proteins.

As used herein, an "O-linked glycan", an "O-glycan" and an "O glycan" are interchangeably used to refer to a glycan which is linked via an oxygen (O) atom or subjected to some modification (for example, acetylation or deacetylation). Typically, the glycan is also called a serine- and threonine-linked glycan since it is linked via OH (a hydroxyl group) of serine or threonine. Examples of such a glycan include O-N-acetylgalactosamine (O-GalNAc) and O-GlcNAc (O-N-acetylglucosamine) produced by an addition reaction of N-acetylgalactosamine to a serine or threonine residue. It is considered that these glycans serve as an indicator for Alzheimer's disease and canceration. It is also known that a gene coding an enzyme capable of removing O-GlcNAc (O-N-acetylglucosamine) is connected to non-insulin-dependent diabetes mellitus and therefore serves as an indicator for diabetes mellitus. Alternatively, the glycans have a function for adhering cells to each other by an interaction between complexes of a large saccharide of proteoglycan and constituting secretory action of mucous membrane. As the O-linked glycan, in addition to this, O-fucose (there are known those in which a consensus sequence of an EGF-like repeat of a Notch protein is added to -C-X-X-G-G-S/T-C- (X is any protein, and fucose is linked toS/T)), O-glucose (there are known those in which a consensus sequence of an EGF-like repeat of a Notch protein is added to -C-X-S-X-P-C- (X is any protein, and glucose is linked to S/T)) and O-mannosyl glycoside also exist. In addition, proteoglycans such as chondroit in sulfate and heparan sulfate are regarded as O-xylosyl glycoside. The "O-linked glycan" as a subject matter of the present invention refers to a glycan separated or produced from a glycan-binding substance having an O-linked glycan by the method of the present invention, and it is understood that an O-linked glycan itself is linked to a glycan-binding substance and those in which have some structural changes (for example, elimination of an acetyl group) are included. Therefore, it is understood that the "O-linked glycan" includes an O-linked glycan and a glycan other than the O-linked glycan in a narrower sense. On the contrary, a glycan linked to an asparagine residue of a protein is called an "N-linked glycan", an "N-glycan", an "N glycan" or an "asparagine-linked glycan". As described above, although the O-linked glycan has important information, no potent enzyme for the O-linked glycan release exists at present and no proper release means exists. Therefore, it can be said that the present invention is excellent in that it enables an automatic glycan-releasing apparatus to release the O-linked glycan and also enables automatic analysis of all glycans.

It is understood that the "glycan-binding substance having an O-linked glycan" as a subject matter of the present inventionmaybe any substance as long as it is a glycan-binding substance having an O-linked glycan, and may be a glycan-binding substance having no other substances linked thereto or a glycan-binding substance having other substances linked thereto, and as a matter of course, it may also be a glycan-binding substance containing an N-linked glycan as long as it has an O-linked glycan. In addition, an O-linked glycan contained in the "glycan-binding substance having an O-linked glycan" and an O-linked glycan originating therein (derived therefrom) are also included. In one aspect, an O-linked glycan contained in the "glycan-binding substance having an O-linked glycan" is exemplified.

In the present description, as the "sample", samples of any origin can be used as long as the sample is intended for separation, concentration, purification or analysis of at least one component (preferably a glycan or a glycan-containing substance) therein. Therefore, samples can be those taken out as a whole or as a portion from living organisms, but are not limited thereto. For example, it is possible to use a sample derived from a body fluid such as serum, a liquid sample such as a cultured cell extract, and a solid sample such as a tissue sample (for example, a formalin-fixed paraffin-embedded(FFPE)tissuesample). In another embodiment, the sample can be one synthesized by a synthetic technique.

As used herein, a "subj ect" refers to an entity containing a target substance in a sample as an analyte of the present invention. The term "test substance" refers to a target substance in a sample as an analyte of the present invention.

As used herein, the term "biological molecule" refers to a molecule that is relevant to the living body. A sample containing such a biological molecule may be sometimes called a "biological sample" herein. As used herein, a "living body" refers to a biological organism and examples thereof include, but are not limited to, animals, plants, fungi, and viruses. Mainly, the test substance as a subject matter of the present invention is often this biological molecule or biological sample, but is not limited thereto. Therefore, the biological molecule includes a molecule to be extracted from the living body, but is not limited thereto, and a molecule capable of exerting an influence on the living body is included in the definition of the biological molecule. Examples of such a biological molecule include, but are not limited to, proteins, polypeptides, oligopeptides, peptides, polynucleotides, oligonucleotides, nucleotides, nucleic acid (for example, including DNA such as cDNA or genomic DNA, and RNA such as mRNA), polysaccharides, oligosaccharides, lipid, low-molecular weight substances (for example, hormones, ligands, information transmitters, and organic low-molecular weight substances), and complex molecules thereof. In the present description, the biological molecule can be preferably those which are expected to contain a glycan-binding substance (for example, glycoprotein or glycolipid).

The supply source of such the biological molecule is not particularly limited as long as it is a material to which a living organism-derived glycan can be linked or attached, and may be any of animals, plants, bacteria and viruses. The supply source is more preferably an animal-derived biological sample. For example, the supply source is preferably whole blood, plasma, serum, sweat, saliva, urea, pancreatic juice, amniotic liquid or cerebrospinal fluid, and more preferably plasma, serum or urea. The biological sample also includes a biological sample which is not previously separated from an individual. For example, epithelium of a mucous membrane tissue which can be brought into contact with a test solution from the outside, or a glandular tissue, preferably a vascular tissue attached to mammary gland, prostate and pancreas is included.

As used herein, "contact" between a glycan-binding substance and an ammonium salt or ammonium ion refers to a state where these two reaction products are brought into close proximity with each other in a level required to undergo a reaction. For example, the contact may be collision between a solid and a solid, or an ammonium salt may be added after being prepared into an aqueous solution to a glycan-binding substance (may be an aqueous solution or a solid), or both the substances may be mixed after being prepared into an aqueous solution. Such contact is preferably performed under the condition in which release of an O-linked glycan from the glycan-binding substance is accelerated. Examples of the condition include the condition in which the ammonium salt is added in an amount ranging from the amount corresponding to half of the saturated concentration to an amount corresponding to the saturated concentration or more, the condition in which the ammonium salt is added in an amount corresponding to the saturated concentration or more, and the condition in which a substance capable of producing an ammonium salt or ammonium ion is added to a solution of the glycan-binding substance in the form of a powder. While not wishing to be bound by any theory, the contact with the ammonium ion in the present invention includes the contact with a hydroxy ion and other basic ions contained in an aqueous ammonium salt solution or the like.

As used herein, "substance capable of producing an ammonium ion" refers to a substance capable of producing an ammonium ion when an aqueous solution is prepared. The substance capable of producing an ammonium ion is typically an ammonium salt, and particularly an aqueous ammonium salt solution, but is not limited thereto. A plurality of substances capable of producing an ammonium ion when mixed (for example, a combination of aqueous ammonia and gaseous carbonic acid) may also be used.

As used herein, "in the absence of concentrated aqueous ammonia" refers to a state when an ammonium salt or ammonium ion is added and where concentrated aqueous ammonia is not substantially added. Therefore, it should be said that mixing of a very small amount of concentrated aqueous ammonia is permitted. Whether or not the concentrated aqueous ammonia is mixed can be determined by a procedure at the time of addition, or judged by the pH at the time of contact with an ammonium salt or ammonium ion, or a relative ratio of an ammonium ion to a counter ion.

This numerical value varies depending on the ammonium salt used. Typically, the pH is about 7 or higher and lower than about 11 and includes, for example, about 7 or higher and lower than about 11, about 7 or higher and about 10 or lower, about 7 or higher and about 9 or lower, about 8 or higher and lower than about 11, about 8 or higher and about 10 or lower, about 8 or higher and about 9 or lower, about 9 or higher and lower than about 11, about 9 or higher and about 10 or lower, and about 10 or higher and lower than about 11. Examples of a preferred range of the pH include about 8.3 or higher and about 10.8 or lower, about 8.5 or higher and about 10.5 or lower, about 8.5 or higher and about 10 or lower, about 9 or higher and about 10 or lower, or about 9.5 or higher and about 9.9 or lower. Regarding these numerical values, the value up to the indicated value is treated as a significant digit. The upper limit is set to about 11 taking into account of the fact that the pH in the standard state when ammonium carbonate is saturated in concentrated aqueous ammonia is about 11 (more accurately 10.98).

As used herein, the indication "about" has the same meaning as the case where there is no such indication unless otherwise indicated specifically, and is interpreted as the indication which permits a variation of ±10% within the range of the significant digit.

In the present invention, in the case of ammonium carbonate, carbonic acid ion : ammonium ion is preferably about 1 : 2 (i.e. ammonium carbonate itself). Alternatively, in the case of ammonium carbamate, carbamic acid ion : ammonium ion is preferably about 1 : 1 (i.e. ammoniumcarbamate itself).

As used herein, "neutralization" refers to removal of an ammonium salt or ammonium ion from the reaction system, thus referring to the fact that the reaction system becomes neutral to weakly acidic (for example, a pH of about 3 to about 5). When the pH is adjusted to about 3 to about 5, a reduced state (aldehyde type) is achieved, and thus analysis is facilitated by BlotGlyco or the like. Therefore, "neutralization" in the present invention is almost the same concept as "separation of an ammonium salt or ammonium ion" from the reaction system. Neutralization can be performed, for example, by using a technique of adding an acid or bringing the reaction system into contact with an ion exchange resin since the ammonium salt or ammonium ion exhibits alkalinity, but the technique is not limited thereto. Examples of the neutralization include a procedure where the solution alkalified by the presence of the ammonium salt or ammonium ion is made neutral to weakly acidic (for example, the pH is from about 4 to 7, e.g. a pH of about 5).

As used herein, "acidify" refers to a procedure where an ammonium salt or ammonium ion is removed from the reaction system and the reaction system becomes weakly acidic (for example, a pH of about 3 to about 5). Thus, glycosylamine is converted into a reducing sugar having a reducing terminal. "Acidification" can be performed, for example, by a technique of adding an acid (for example, acetic acid, formic acid, or trifluoroacetic acid) or bringing the reaction system into contact with an ion exchange resin since the ammonium salt or ammonium ion exhibits alkalinity, but the technique is not limited thereto. Examples of the acidification include turning the solution alkalified by the presence of the ammonium salt or ammonium ion to weakly acidic (for example, the pH is from about 3 to about 5). In one embodiment, the reaction solution obtained by treatment with the ammonium salt or ammonium ion can be kept warm (or treated) under an acidic condition after neutralization. Preferably, the reaction solution is made acidic by neutralization. While not wishing to be bound by any theory, when ammonia carbonate is quickly acidified with a large amount of an acid, a large amount of carbon dioxide may be generated at a time to cause drastic bubbling, resulting in sample loss.

As used herein, "collection" of a glycan refers to obtaining the released glycan after the reaction. As long as the glycan can be collected, any technique can be used. Examples of such a technique include gel filtration, various chromatographies such as ion exchange chromatography and affinity chromatography, high-performance liquid chromatography (HPLC), and glycan purification using beads for glycan immobilization.

### (Separation and Production of Glycan)

In one aspect, the present invention provides a method of, a kit for, or a device for producing or separating an O-linked glycan from a glycan-binding substance having the O-linked glycan.

The technology of the present invention is a method of producing or separating an O-linked glycan from a glycan-binding substance having the O-linked glycan, the method includes the steps of (A) bringing an ammonium salt or ammonium ion into contact with the glycan-binding substance under the condition in which the pH is about 7 or higher and lower than about 11; (B) removing the ammonium salt or ammonium ion from the reaction system obtained in step (A) (for example, neutralizing the reaction solution obtained in step (A), and optionally acidifying the solution); and (C) collecting the released glycan. If necessary, the collected glycan is further analyzed. Examples of such a substance include, but are not limited to, glycoprotein, glycopeptide, proteoglycan, glycosaminoglycan, glycolipid, and carbohydrate-nucleic acid complex. In the method of the present invention, examples of the glycan-binding substance having an O-linked glycan as a subject matter include a substance containing serine or threonine (for example, glycopeptide). This is because the O-linked glycan is usually linked to serine or threonine.

In conventional art, although the method of retaining a reducing terminal exists, the peeling reaction cannot be suppressed. Even if the suppression is possible, since a potent reducing agent is used, the reducing terminal is converted into an alditol, resulting in drawbacks such as restriction on the subsequent sample handling. Here, the peeling reaction refers to the decomposition of the reducing terminal under the conditions such as an alkaline condition, resulting in the decomposition of polysaccharides. More specifically, isosaccharic acid and metasaccharic acid terminal groups are produced from the terminal group of 1-4-linked polysaccharides under the alkaline conditions through a fructose type. That is, it is a reaction in which a saccharide residue is eliminated from the reducing end group one by one through a β-alkoxycarbonyl elimination reaction, and thus the polymerization degree decreases. While not wishing to be bound by any theory, it is difficult in conventional art to simultaneously perform the step of releasing a saccharide and increasing the efficiency, and suppressing the peeling reaction and thus, it has not been achieved. It should be said that the present invention exerts a remarkable effect in the respect that this was achieved by using the steps of the present invention in combination.

In addition, Patent Document 1 proposes a method utilizing concentrated aqueous ammonia. However, operation and handling is difficult since concentrated aqueous ammonia is used, and it is also difficult to construct a system for automation of glycan analysis. The present invention provides a glycan releasing method which can be applied to the system construction for automation of glycan analysis, particularly a glycan releasing method capable of analyzing an O-linked glycan, thus making it possible to realize an all-in-one automatic glycan analysis system.

First, the present invention has a feature that an ammonium salt or ammonium ion is brought into contact with a glycan-binding substance containing an O-linked glycan in the absence of concentrated aqueous ammonia or under the condition in which the pH is about 7 or higher and lower than about 11, or an ammonium salt, particularly an aqueous ammonium solution is added to a glycan-binding substance containing an O-linked glycan. Such a feature has an advantage that undesired side reaction exerted by concentrated aqueous ammonia solution, which is considered to be essential in Patent Document 1, can be eliminated.

It is possible to use, as the ammonium salt or ammonium ion which can be used in the present invention, any ammonium salt and an ammonium ion derived therefrom. Examples thereof include ammonium chloride, ammonium hydrogen citrate, ammonium carbamate, ammonium bicarbonate, ammonium carbonate, and tetrabutylammonium hydroxide, and these substances can be used alone or in combination. Preferably, ammonium carbamate, ammonium bicarbonate and ammonium carbonate are used. Particularly preferably, ammonium carbamate and ammonium carbonate are used.

Alternatively, in the present invention, it is also possible to use a nitrogen (N)-containing substance such as an analogous amine or piperidine in place of the ammonium salt or ammonium ion. Examples of such an amine include piperidine, triethylamine, tributylamine, N,N-diethylaniline, and diethylamine.

Such an ammonium salt or ammonium ion, or an analogous nitrogen (N)-containing substance may be added under any condition as long as an O-linked glycan can be separated. Examples of such a condition include, but are not limited to, the condition in which the ammonium salt is added in an amount corresponding to 0.01 M or more, 1/100 or more of the saturated concentration, 0.1 M or more, 1/10 or more of the saturated concentration, 0.5 M or more, or an amount ranging from the amount corresponding to half of the saturated concentration to an amount corresponding to the saturated concentration or more, and preferably the condition in which the ammonium salt is added in an amount corresponding to the saturated concentration or more.

More specifically, where a preferred ammonium salt such as ammonium carbamate, ammonium bicarbonate or ammonium carbonate is used, it is possible to use the condition in which the ammonium salt is added in an amount ranging from the amount corresponding to half of the saturated concentration to the amount corresponding to the saturated concentration or more, and preferably the condition in which the ammonium salt is added in an amount corresponding to the saturated concentration or more.

The pH condition when using such an ammonium salt or ammonium ion or an analogous N-containing substance may be any condition as long as the pH is lower than 11 taking into account of the condition that concentrated aqueous ammonia is absent. Preferably, the pH condition is neutral to alkaline. For example, the pH condition varies depending on the ammonium salt used. In a preferred embodiment, ammonium salts of weak acids, such as ammonium carbamate, ammonium bicarbonate and ammonium carbonate are used. Therefore, the pH is typically about 7 or higher and lower than about 11, for example, about 7 or higher and about 10 or lower, about 7 or higher and about 9 or lower, about 8 or higher and lower than about 11, about 8 or higher and about 10 or lower, about 8 or higher and about 9 or lower, about 9 or higher and lower than about 11, about 9 or higher and about 10 or lower, or about 10 or higher and lower than about 11. Examples of the lower limit include numerical values such as about 8.0, about 8.1, about 8.2, about 8.3, about 8.4, about 8.5, about 8.6, about 8.7, about 8.8, about 8.9, about 9.0, about 9.1, about 9.2, about 9.3, about 9.4, about 9.5, about 9.6 and about 9.7. Examples of the upper limit include about 9.7, about 9.8, about 9.9, about 10.0, about 10.1, about 10.2, about 10.3, about 10.4, about 10.5, about 10.6, about 10.7, about 10.8, about 10.9, and less than about 11.0. Preferred ranges can be about 8.3 or higher and about 10.8 or lower, about 8.5 or higher and about 10.5 or lower, about 8.5 or higher and about 10 or lower, about 9 or higher to about 10 or lower, or about 9.5 or higher and about 9.9 or lower. In the presence of concentrated aqueous ammonia, for example, in the case of saturated ammonium carbonate, since the pH is about pH 11 (more accurately 10.98) in a standard state, it is understood that the pH value lower than the above value can be used in the present invention. As a matter of course, since the pH varies depending on the measurement condition, it is understood that a person ordinary skilled in the art can understand the variation and can take it into consideration when using the present invention.

The duration of time to contact such an ammonium salt or ammonium ion or an analogous N-containing substance (or a treatment duration, for example, the time from the addition of an ammonium salt (particularly, an aqueous ammonium salt solution) to the subsequent treatment) may be a duration enough to release an O-linked glycan in the intended level, and examples thereof include 10 hours or more, 20 hours or more, and 40 hours or more. The upper limit is not particularly decided as long as side reactions such as other decomposition reactions do not occur. From the viewpoint of efficiency of the operation, the upper limit is preferably up to about 60 hours, for example, a duration from 20 hours to 40 hours can be used.

The temperature at which such an ammonium salt or ammonium ion or an analogous N-containing substance is treated may be the temperature sufficient for releasing an O-linked glycan in the intended level and may be, for example, from 0°C to room temperature. Preferably, it is advantageous to heat the reaction system. While not wishing to be bound by any theory, it is considered that a hydrolysis reaction of the O-linked glycan satisfactorily proceeds by heating. While not wishing to be bound by any theory, when the temperature is too high, a side reactionmay arise. Therefore, the temperature is preferably 100°C or lower and can be, for example, 40°C to 80°C, 40°C to 60°C or 60°C to 80°C, and the treatment is preferably carried out at 60°C. While not wishing to be bound by any theory, the temperature to be adopted here does not have to be closely kept at 60°C, as long as it may be the temperature at which an ammonium salt is not decomposed, and a temperature of about 60°C may be appropriately adopted since all of ammonium carbamate, ammonium bicarbonate and ammonium carbonate do not decompose into water, carbon dioxide and ammonia at 60°C or higher.

The concentration of a glycan-binding substance as a subject matter of the method of the present invention may be any concentration.

Examples of the method of neutralizing such an ammonium salt or ammonium ion or an analogous N-containing substance include addition of an acid and use of an ion exchange resin.

It is possible to utilize, as the acid to be used, any acid as long as such an ammonium salt or ammonium ion or an analogous N-containing substance can be neutralized. Preferably, the acid is an acid which does not cause deterioration of the produced glycan. Any acid can be utilized as long as it is an inorganic or organic acid which is usually used. Preferably, a weak acid is used. This is because it is easy to control neutralization of the ammonium salt or ammonium ion. Examples of such an acid include acetic acid, formic acid, trifluoroacetic acid, and boric acid. Preferably, acetic acid can be used. While not wishing to be bound by any theory, the reason why acetic acid, formic acid, and trifluoroacetic acid are preferably used is that they do not cause deterioration of the produced glycan and that the salt produced at the time of neutralization is volatile. It is possible to use an acid having an appropriate concentration (for example, the concentration may be 17.4 M, but is not limited thereto).

It is possible to use, as an ion exchange resin used for neutralization in the present invention, any ion exchange resin as long as it can separate or neutralize an ammonium salt or ammonium ion or analogous N-containing substance. Examples of such an ion exchange resin include a cation exchange resin under the trade name of DOWEX50 (H+).

In the present invention, one or both of the acid and the ion exchange resin may be used.

In the step of collecting a glycan used in the present invention, any technique can be used as long as the released glycan can be collected. Examples of such a technique include gel filtration, various chromatographies such as ion exchange chromatography and affinity chromatography, high-performance liquid chromatography (HPLC), and glycan purification using beads for glycan immobilization. Preferably, it is possible to achieve the collection by an existing method using BlotGlyco beads (also referred to as a "glycoblotting method"; Furukawa J-I. et al., Anal. Chem., 80, 1094-1101, 2008, Miura Y. et al., Mol. Cell. Proteomics, 2008 Feb; 7 (2) : 370-7. Epub 2007 Nov 5.). It is also possible to use beads having an aminooxy group, which have the same concept as BlotGlyco beads, or hydrazide beads such as AffiGel Hz and BioRad.

The "glycoblotting method" used in the present description can be carried out as follows.

A released glycan having a reducing terminal is linked onto a solid phase (beads) having an aminooxy group or a hydrazide group and, after a treatment such as washing or sialic acid modification, the glycan is collected from the solid phase using an acid or any aminooxy compound or hydrazide compound.

In such a technique, for example, in a glycan purification method using beads for glycan immobilization, the reaction solution is applied to beads for glycan immobilization (for example, BlotGlyco beads, AffiGel Hz) and the released glycan is linked thereto and, after a treatment such as washing or sialic acid modification, the glycan is collected. Alternatively, such a technique is achieved by making a graphitized carbon resin (CarboGraph cartridge, etc.) adsorbing the reaction solution, followed by washing and further eluting the solution with an eluate (for example, 25% acetonitrile/0.05% trifluoroacetic acid).

In a preferred embodiment, the present invention provides a method of producing an O-linked glycan from a glycan-binding substance containing the O-linked glycan, the method includes the steps of (A) adding an ammonium bicarbonate, ammonium carbonate or ammonium carbamate powder or an aqueous solution of an ammonium bicarbonate, ammonium carbonate or ammonium carbamate powder to an aqueous solution containing the glycan-binding substance so that the aqueous solution satisfies the half saturation to saturation condition, or preferably adding an ammonium bicarbonate, ammonium carbonate or ammonium carbamate powder or an aqueous solution of an ammonium bicarbonate, ammonium carbonate or ammonium carbamate powder so that the aqueous solution satisfies the half saturation to saturation condition, followed by heating to about 60°C; (B) neutralizing the reaction solution obtained in step (A) with acetic acid, formic acid, trifluoroacetic acid or boric acid, preferably acetic acid, formic acid, trifluoroacetic acid (more preferably acetic acid) (and also acidifying the reaction solution with acetic acid, formic acid, trifluoroacetic acid or boric acid, preferably acetic acid, formic acid, trifluoroacetic acid (more preferably acetic acid) (thereby adjusting the pH to preferably about 3 to about 5)); and (C) collecting the released glycan using a glycan capturing carrier, for example, BlotGlyco. It has already been demonstrated by the present invention that the O-linked glycan can be efficiently separated by these methods, and also can be quantitatively determined.

In another more preferred embodiment, the present invention provides a method of detecting an O-linked glycan, the method includes the steps of (A) adding an ammonium bicarbonate, ammonium carbonate or ammonium carbamate powder or an aqueous solution of an ammonium bicarbonate, ammonium carbonate or ammonium carbamate powder to an aqueous solution containing the glycan-binding substance so that the aqueous solution satisfies the half saturation to saturation condition, or preferably adding an ammonium bicarbonate, ammonium carbonate or ammonium carbamate powder or an aqueous solution of an ammonium bicarbonate, ammonium carbonate or ammonium carbamate powder so that the aqueous solution satisfies the half saturation to saturation condition, followed by heating to about 60°C; (B) acidifying the reaction solution obtained in step (A) with acetic acid, formic acid, trifluoroacetic acid or boric acid, preferably acetic acid, formic acid or trifluoroacetic acid (more preferably acetic acid) ; and (C) collecting the released glycan using a glycan capturing carrier, for example, BlotGlyco, and analyzing the released glycan by mass spectrometry (for example, MALDI-TOFMS).

In another aspect, the present invention provides a composition for producing or separating an O-linked glycan from a glycan-binding substance having the O-linked glycan, the composition containing ammonium carbonate, ammonium bicarbonate or ammonium carbamate, wherein when the ammonium carbonate is selected, the ammonium carbonate exists in the absence of concentrated aqueous ammonia. Preferably, the present invention provides a composition for producing or separating an O-linked glycan from a glycan-binding substance having the O-linked glycan, the composition containing ammonium bicarbonate or ammonium carbamate. An ammonium salt or ammonium ion used in this composition of the present invention can exist under any condition described in the present description. The composition of the present invention was not known in the use for producing or separating an O-linked glycan from a glycan-binding substance having the O-linked glycan. It was also not known that ammonium carbonate is used in the absence of concentrated aqueous ammonia. Therefore, the present invention provides a use which has not been known conventionally.

In another aspect, the present invention provides a kit for producing or separating an O-linked glycan from a glycan-binding substance having the O-linked glycan, the kit includes (A) an ammonium salt or ammonium ion, (B) means for neutralizing (and/or means for acidifying) the ammonium salt or ammonium ion and (C) means for collecting a glycan. It is possible to use, as the ammonium salt or ammonium ion, the neutralizing means (forexample, an acid or an ion exchange resin), the acidifying means (for example, an acid or an ion exchange resin) and the means for collecting a glycan (for example, a glycan capturing carrier), any embodiment used in the present description.

### (Analysis of Glycan)

As used herein, "analysis" of a glycan refers to examining the kind, structure, linkage type inside the glycan, amount, and linkage type in a glycan-binding substance such as a protein of the glycan qualitatively and quantitatively.

In the analysis of the separated molecule, an appropriate method can be used according to the kind of the target molecule and, for example, mass spectrometry (MS) and/or nuclear magnetic resonance (NMR) can be used. It is possible to use, in addition to mass spectrometry (MS) and/or nuclear magnetic resonance (NMR), ultraviolet spectrometry (UV), evaporative light scattering detector (ELS), electrochemical detector (particularly to a glycan and glycopeptide), liquid chromatography-mass spectrometry method and the like. It is also possible to determine the structure by using these methods in combination with a glycosidase. In the present invention, released N-GalNAc, N-GlcNac, O-mannose and O-fucose glycans can be analyzed.

The technology of mass spectrometry used in the method of the present invention is well known in the relevant technical filed and, for example, it is possible to refer to Niwa, Latest Mass Spectrometry, Kagaku-Dojin Publishing Company, Inc., 1995; Modern NMR Spectroscopy: A guide for Chemists, J. K. M. Sanders and B. K. Hunter (2nd Ed., Oxford University Press, New York, 1993); Spectrometric Identification of Organic Compounds, R. M. Silverstein, G. Clayton Bassler, and Terrence C. Morill (5th Ed., John Wiley & Sons, New York, 1991) and the like. In mass spectrometry in the method of the present invention, it is possible to utilize any mass spectrometer using any ionization technique (for example, an electrospray (ESI) method or a matrix-assisted laser desorption/ionization (MALDI) method) which is commonly utilized in the relevant technical filed. It is possible to use any mass separating system (for example, a Time-of-Flight mass spectrometer, a quadrupole mass spectrometer, or a magnetic sector mass spectrometer) for mass spectrometry in the method of the present invention. In a preferred embodiment, mass spectrometry is performed by MALDI-TOF MS.

In one preferred example, mass spectrometry of the O-linked glycan separated by the method of the present invention can be performed, for example, by MALDI-TOF MS (for example, Ultraflex and Biflex are available from Bruker Corp.).

Preferably, a matrix reagent is used in the mass spectrometry. Any matrix reagent can be used as long as it is commonly used in mass spectrometry, and it is preferably a reagent substantially free from a substance having a keto group. The reason is that a reaction between an aldehyde group in a fluid and the substance of the present invention does not sufficiently proceed when a significant amount of a substance having a keto group is present. Therefore, the sample to be subjected to mass spectrometry in a preferred embodiment is free from a substance having a keto group. Examples of a preferred matrix reagent include, but are not limited to, 2,5-dihydroxybenzoic acid, α-cyano-4-hydroxy-cinnamic acid, sinapic acid, trans-3-indole-acrylic acid, 1,5-diamino-naphthalene, 3-amino-4-hydroxybenzoic acid, 9-nitro-anthracene, 2-picolinic acid, 3-hydroxy-picolinic acid, nicotinic acid, anthranilic acid, 5-chloro-salicylic acid, 2'-(4-hydroxyphenylazo)benzoic acid, dithranol and 3-amino-quinoline. In a more preferred embodiment, the matrix reagent is 2,5-dihydroxybenzoic acid (available from Fluka Corp.).

In one embodiment, by adding a reagent capable of adding a quaternary amine, such as Girard T (manufactured by Sigma), to the obtained glycan, signal sensitivity of the glycan in MALDI-TOF can be increased. However, the reagent is not limited thereto.

Such an analysis technique of the glycan, which can be utilized in the present invention, can be carried out by referring to PCT International Publication Pamphlets WO 2004/058687, WO 2006/0305841, WO 2009/044900 and the like.

Examples of the method of analyzing a glycan or a glycan-containing substance in a sample includes a method including the steps of: (a) bringing a glycan capturing carrier containing a substance (i.e. a glycan capturing substance) capable of specifically interacting with a glycan in a fluid phase into contact with the sample under the condition in which the glycan capturing carrier can be reacted with the glycan; (b) exposing the glycan capturing carrier and the sample under the condition of the desired stringency (i.e. the condition in which an interaction between a substance capable of specifically interacting with a glycan and the glycan or a glycan-containing substance does not undergo dissociation. A person ordinary skilled in the art can appropriately select such a condition taking various parameters such as a reagent, a carrier, a glycan or a glycan-containing substance to be used, a substance which specifically interacts with the glycan, and an interaction to be formed into consideration using a technology well known in the relevant technical filed. For example, in case the interaction is a covalent linkage, the desired stringency may be rinsing with water (for example, ultrapure water) or a buffer (for example, an acetate buffer)); and (c) identifying the substance interacted with the glycan capturing carrier.

The separated O-linked glycan can be labeled. Such labeling can be achieved by setting a condition in which a labeled compound can react with this glycan. Examples of such a condition include a condition in which a functional group, which specifically reacts with an aldehyde group, specifically reacts with an aldehyde group of a glycan. This reaction condition can be appropriately selected by a person ordinary skilled in the art by appropriately setting the parameters such as the reaction temperature, the reaction time, the concentration of a labeled compound and a subject compound, the reaction medium (a fluid such as a solvent or a matrix solution), the reaction vessel, the pH, the salt concentration and the pressure. Regarding these parameters, JP-A-2005-291958 can be referred to.

The glycan capturing substance used in this reaction is typically a polymer having an aminooxy group or a hydrazide group, and these reactive functional groups react with an aldehyde group in the equilibrium between a cyclic hemiacetal and a non-cyclic aldehyde formed from a glycan in a solution such as an aqueous solution to form a specific and stable linkage, thus making it possible to capture the glycan.

When a liquid chromatography-mass spectrometry is used, a sample containing a neutral glycan and an acidic glycan is delivered to a separation column by an eluant (pH 3 to 5) and the sample is separated into respective components by the separation column. The sample separated into the respective components is delivered to an ion source capable of ionization by spraying at a high rate and this ion source is operated by a negative ion measuring mode thereby ionizing the sample, and thus mass spectrometry of the ionized sample can be performed.

The sample eluted from separation means for separating the sample into respective components is ionized and ions having any mass number are cleaved. In a mass spectrometer using a mass spectrometry or tandem mass spectrometry (MSn) technology, using databases in which correlation information between an isomer abundance ratio and a specific ionic intensity ratio in a mass spectrum is stored for each isomer, it is possible to determine whether or not an isomer is contained in the sample using each database. When isomers are contained, mass spectrometry can be achieved by calculating an abundance ratio between isomers.

Here, a glycan capturing reaction, namely a reaction between the glycan capturing substance and the biological sample, which has already been subjected to a treatment by the present invention, is performed by introducing the glycan capturing substance into the pretreated sample in a reaction system under the condition of an acidic pH, preferably the condition in which the pH is from 2 to 6, and more preferably from 3 to 6, and under the condition in which the reaction temperature is from 4 to 90°C, preferably from 25 to 90°C, and more preferably from 40 to 90°C for 10 minutes to 24 hours, preferably 10 minutes to 8 hours, and more preferably 10 minutes to 2 hours.

In another aspect, the present invention provides a system used for detecting an O-linked glycan in a sample which is expected to contain a glycan-binding substance having the O-linked glycan, the system includes: (A) an ammonium salt or ammonium ion, (B) means for neutralizing the ammonium salt or ammonium ion (and optionally acidifying the ammonium salt or ammonium ion), and (C) means for detecting a glycan. It is possible to use, as the ammonium salt or ammonium ion, the neutralizing means (for example, an acid or an ion exchange resin), the acidifying means (for example, an acid or an ion exchange resin) and means for analyzing a glycan (for example, MALDI-TOF) used in this system of the present invention, any embodiment described in the present description. This system may include means for collecting a glycan (for example, a glycan capturing carrier).

### (Detection and Diagnosis)

In another aspect, the present invention provides a method in which a disease related to an O-linked glycan is analyzed utilizing a method of detecting the O-linked glycan of the present invention, thereby specifying an etiology and making a diagnosis. Such a diagnostic method can be determined with reference to a known medical knowledge based on the data about the once separated O-linked glycan.

As used herein, "detection" refers finding a peak to be recognized as a signal in a spectrum to be observed, for example, in the context of mass spectrometry. In case the peak which is the recognized subject matter corresponds to a specific subject, it is said that the peak of the subject is detected. Alternatively, in the context related to diagnosis, "detection" refers to identify various parameters related to disease, disorder, etiology, condition and the like in the subject.

As used herein, "diagnosis" refers to identification of various parameters related to disease, disorder, etiology, condition and the like in the subject and then judgment of the present state of the disease, disorder and condition. By using the method, device and system of the present invention, a glycan can be identified, and various parameters such as disease, disorder, etiology and condition in the subject can be selected using information about the identified glycan. As used herein, "diagnosis" includes the concept of "discrimination" capable of identifying various parameters such as disease, disorder, etiology and condition in the subject.

According to the present invention, etiology can be examined and diagnosed by analyzing an O-linked glycan.

As used herein, "etiology" refers to a factor involved in disease, disorder or condition (generally called "pathological change" herein and also called lesion in plants) of the subject, and examples thereof include, but are not limited to, causative pathogenic substances (pathogenic factors), pathogens, pathological cells, and pathogenic viruses.

Examples of such a disease, disorder or condition include, but are not limited to, circulatory system diseases (anemia (for example, aplastic anemia (particularly, serious aplastic anemia), renal anemia, cancerous anemia, secondary anemia, and refractory anemia), and cancers and tumors (for example, leukemia and multiple myeloma), etc.); nervous system diseases (dementia, cerebrovascular accident and after effects thereof, braintumor, spinal cord injury, etc.); immune system diseases (T-cell depletion, leukemia, etc.); locomotorium and skeletal diseases (bone fracture, osteoporosis, joint dislocation, subluxation, sprain, ligament injury , degenerative arthritis, osteogenic sarcoma, Ewing's sarcoma, osteogenesis imperfecta, osteochondrodysplasia, etc.); skin diseases (atrichia, melanoma, cutaneous malignant lymphoma, angiosarcoma, histiocytosis, blister, pustulosis, dermatitis, eczema, etc.); endocrine diseases (hypothalamo-pituitary disease, thyroid disorder, parathyroid (glandula parathyroidea superior) disease, adrenal cortex-medulla disease, anomaly of saccharometabolism, lipidosis, anomaly of protein metabolism, anomaly of nucleic acid metabolism, inborn error of metabolism (phenylketonuria, galactosemia, homocystinuria, maple syrup urine), analbuminemia, missing ascorbic acid biosynthesis, hyperbilirubinemia, bilirubinuria, deficiency of components of kallikrein, mast cell deficiency, diabetes insipidus, inappropriate vasopressin secretion, dwarfism, Wolman's disease (Acidlipase deficiency), mucopolysaccharidosis VI, etc.); respiratory diseases (lung diseases (for example, pneumonia and lung cancer), bronchial disease, lung cancer, bronchial cancer, etc.); digestive system diseases (esophageal diseases (for example, esophageal cancer), stomach and duodenal diseases (for example, gastric cancer and duodenal cancer), small intestinal diseases and large intestinal diseases (for example, colonic polyp, colon cancer, and rectal cancer), biliary tract diseases, liver diseases (for example, hepatic cirrhosis, hepatitis (type A, B, C, D, E, etc.), hepatitis fulminant, chronic hepatitis, primary hepatic cancer, hepatopathy alcoholic, and drug-induced hepatic injury), pancreas diseases (acute pancreatitis, chronic pancreatitis, pancreas cancer, cystic pancreatic disease, etc.), peritoneum-abdominal wall-diaphragm diseases (hernia, etc.), Hirschsprung's disease, etc.); urinary system diseases (kidney diseases (renal insufficiency, primary glomerular disease, renovascular disease, renal tubular dysfunction, interstitial renal disease, renal damage due to systemic disease, renal cancer, etc.), urinarybladder diseases (inflammation of the bladder, bladder cancer, etc.), etc.); reproductive system diseases (male genital diseases (male infertility, prostatic hypertrophy, prostate cancer, testicular cancer, etc.), female genital diseases (female infertility, ovarian function disorder, uterine myoma, adenomyosis of the uterus, uterine cancer, endometriosis, ovarian cancer, trophoblastic disease, etc.), etc.); circulatory system diseases (cardiac failure, angina, myocardial infarction, cardiac arrhythmia, valvular disease, myocardial and pericardial disease, congenital heart diseases (for example, atrial septal defect, interventricular septal defect, patent ductus arteriosus, and Fallot's tetralogy), arterial diseases (for example, arteriosclerosis and aneurysm), venous diseases (for example, varices), lymphatic vessel diseases (for example, lymphoedema), etc.) and the like.

As described above, the present invention can be applied to, in addition to medical care, all of those which require an inspection of biological molecules in food inspection, quarantine inspection, drug inspection, forensic medicine, agriculture, stock raising, fishery, forestry and the like. In the present invention, particularly, use for food safety purposes (for example, BSE inspection) is also intended.

The present invention can also be used for detection of various glycans, and can also be used for various inspections, diagnoses, judgments and discriminations since the kind of the glycan to be detected is not particularly limited. It is possible to use the present invention for detection of the glycan which is specific to genes of viral pathogens (including, but are not limited to, hepatitis viruses (type A, B, C, D, E, F, G, etc.), HIV, influenza viruses, herpes group viruses, adenoviruses, human polyomaviruses, human papillomaviruses, human parvoviruses, mumps viruses, human rotaviruses, enteroviruses, Japanese encephalitis viruses, dengue viruses, rubella viruses and HTLV) ; genes of bacterial pathogens (including, but are not limited to, Staphylococcus aureus, hemolytic streptococcus, enteropathogenic Escherichia coli, Vibrio parahaemolyticus, Helicobacter pylori, campylobacter, cholera vibrio, dysentery bacillus, salmonella, Yersinia, gonococci, listeria, Leptospira, Legionella, spirochete, Mycoplasma pneumoniae, rickettsia and chlamydia); malaria, dysentery amoeba, pathogenic fungus, parasitic worm, fungus and the like.

Alternatively, the present invention can also be used for detection of data obtained from biochemical inspection. Examples of the item of the biochemical inspection include, but are not limited to, data items which are considered to be involved in glycans such as cholinesterase, alkaline phosphatase, leucine aminopeptidase, γ-glutamyl transpeptidase, creatine phosphokinase, lactic dehydrogenase, and amylase.

As described above, the method, device and system of the present invention can be used in, for example, diagnosis, forensic medicine, drug seeking (drug screening) and development, molecular biological analysis (for example, array-based glycan analysis), analysis of glycan characteristics and functions, pharmacology, glycomics, environmental research and further biological and chemical analysis.

### (Screening)

As used herein, "screening" refers selecting substances or living organisms having the intended certain specific property from a lot of candidates by a specific operation and/or evaluation method. In the present description, screening can be performed by knowing the kind, amount, abundance ratio, linkage type and the like utilizing the method of detecting an O-linked glycan of the present invention. In the present invention, it is understood that compounds obtained by screening having the desired activity are also included within the scope of the present invention. In the present invention, it is also intended to provide a drug resulting from computer modeling based on the disclosure of the present invention.

### (Well-Known Technology)

As the technology used in the present description, for example, well-known conventional technologies in the fields of analytic chemistry, organic chemistry, biochemistry, genetic engineering, molecular biology, microbiology, genetics and related fields thereof, which are within the technical scope of the relevant technical filed, are used unless otherwise specified. These technologies are sufficiently explained, for example, in literatures listed below, and literatures cited in other passages of the present description.

Organic chemistry is described, for example, in Morrison Boyd Organic Chemistry, First, Second and Final Volumes, 5th edition (published by Tokyo Kagaku Dozin (1989)), March, Advanced Organic Chemistry, 4th edition (Wiley Intersience, JOHN WILEY & SONS, 1992) and the like, and related passages are incorporated by reference herein.

A molecular biological technique, a biochemical technique and a microbiological technique used herein are well-known in the relevant technical filed and conventionally used and are described, for example, in Maniatis, T. et al. (1989). Molecular Cloning: A Laboratory Manual, Cold Spring Harbor and the 3rd Ed. Thereof (2001); Ausubel, F. M., et al. eds, Current Protocols in Molecular Biology, John Wiley & Sons Inc., NY, 10158 (2000); Innis, M.A. (1990). PCR Protocols: A Guide to Methods and Applications, Academic Press; Innis, M. A. et al. (1995). PCR Strategies, Academic Press; Sninsky, J. J. et al. (1999). PCR Applications: Protocols for Functional Genomics, Academic Press; Gait, M. J. (1985) . Oligonucleotide Synthesis: A Practical Approach, IRL Press; Gait, M. J. (1990). Oligonucleotide Synthesis: A Practical Approach, IRL Press; Eckstein, F. (1991). Oligonucleotides and Analogues: A Practical Approach, IRL Press; Adams, R. L. et al. (1992). The Biochemistry of the Nucleic Acids, Chapman & Hall; Shabarova, Z. et al. (1994). Advanced Organic Chemistry of Nucleic Acids, Weinheim; Blackburn, G. M. et al. (1996). Nucleic Acids in Chemistry and Biology, Oxford University Press; Hermanson, G. T. (1996). Bioconjugate Techniques, Academic Press; Method in Enzymology 230, 242, 247, Academic Press, 1994;Bessatsu Jikken Igaku "Gene Transfer & Expression Analytical Test Method" YODOSHA CO., LTD., 1997; Hatanaka, Nishimura et al., Science and Engineering of Glucide, Kodansha Scientific Corporation, 1997; Design and Physiological Mechanism of Glycan Molecule, edited by The Chemical Society of Japan, Japan Scientific Societies Press, 2001 and the like, and related passages (the entire contents of which may be available) are incorporated by reference herein.

The entire contents of references such as scientific literatures, patents and patent applications cited herein are incorporated by reference herein to the same extent as each one is specifically described.

While preferred embodiments of the present invention have been described and illustrated above for easier understanding, the present invention will be described below based on examples, and such are for illustrative purposes only and should not be construed restrictively. Therefore, the scope of the present invention is not limited by the foregoing embodiments and examples described specifically in the present specification, but is only limited by the scope of the appended claims.

### EXAMPLES

The present invention will be described below by way of examples including experimental examples. However, the present invention is not limited thereto.

### (Example 1: Examination of Release of O-Linked Glycan from Glycoprotein by Various Basic Substances)

Release of an O-linked glycan from glycoprotein (bovine submaxillary mucin, also abbreviated as "BSM" in this specification) by various basic substances was confirmed by mass spectrometry, and thus an effective glycan release method was examined.

BSM was dissolved in ultrapure water in the concentration of 40 mg/mL and 0.8 mg of the obtained solution was used in each experiment. The reaction conditions were as follows: a treatment time of 20 hours and a treatment temperature of 60°C, and a treatment was performed in duplicate. In the examination of various ammonium salts, a treatment was performed at a saturated or half saturated salt concentration. An organic amine group was dissolved in methyl alcohol in the concentration of 1 M and the obtained solution was mixed with an equivalent amount of a protein solution, followed by a reaction at 0.5 M.

### (Examined Reagents)

Ammonium salts used for examination are as follows:
Ammonium carbonate,
Ammonium hydrogen carbonate,
Ammonium formate,
Ammonium chloride,
Diammonium hydrogen citrate,
Ammonium carbamate, and
Tetrabutylammonium hydroxide solution.
Organic amines used for examination are as follows:
Piperidine,
Triethylamine,
Tributylamine,
N,N-diethylaniline, and
Diethylamine.

### (Measurement of pH)

Ammonium chloride (Wako Pure Chemical Industries, Ltd. : ammonium chloride NH₄Cl 53.49), ammonium bicarbonate NH₄HCO₃ (Sigma: Ammonium bicarbonate NH₄HCO₃ 79.06), ammonium carbonate (Wako Pure Chemical Industries, Ltd.: ammonium carbonate (NH₄)₂CO₃ 96.086) and ammonium carbamate (Tokyo Chemical Industry Co., Ltd.: ammonium carbamate NH₂COONH₄ 78.07) were saturated in 10 mL of ultrapure water at normal temperature, and then the pH was measured within a range from 21°C to 23°C using a pH meter manufactured by HORIBA Ltd. (HORIBA pH METER F-22). As a comparative example, ammonium carbonate was saturated in concentrated aqueous ammonia and then the similar measurement was performed.

### (Post Reaction Treatment)

After each reaction, the pH was adjusted within a range from about 4 to about 5 using acetic acid, and 2.5 nmol of chitotetraose (GN4) was added as an internal standard.

Ultrapure water was added to the obtained solution to make 100 µL. Using 40 µL of the solution in a glycoblotting method using BlotGlyco beads, the released glycan was purified and collected. The collected sample was analyzed by mass spectrometry and the release of a glycan was confirmed. In the collected glycan, the sialic acid moiety was converted into a methyl ester, mass spectrometry was quantitatively performed in a "positive mode".

### (Results)

The amount of the released collected glycan under each condition was shown in Fig. 1 as a relative amount to GN4 added as an internal standard. The numerical value was as follows. NeuAc represents N-acetylneuraminic acid, and NeuGc represents N-glycolylneuraminic acid. HexNAc denotes N-acetylhexosamine.

[Table 1]

**Table 1**

| | Saturated ammonium carbonate (Relative intensity) | Saturated ammonium carbamate (Relative intensity) | Half saturated ammonium carbonate (Relative intensity) | Half saturated ammonium hydrogen carbonate (Relative intensity) | Half saturated ammonium carbamate (Relative intensity) |
|---|---|---|---|---|---|
| (HexNAc)₁ (NeuAc)₁ | 1.65 | 1.76 | 1.68 | 0.77 | 2.02 |
| (HexNAc)₁ (NeuGc)₁ | 0.80 | 0.88 | 0.87 | 0.36 | 1.02 |
| (HexNAc)₂ (NeuAc)₁ | 1.89 | 1.86 | 1.24 | 0.57 | 1.86 |
| (HexNAc)₂ (NeuGc)₁ | 1.10 | 1.47 | 0.67 | 0.29 | 1.03 |
| (HexNAc)₄ | 1.00 | 2.00 | 1.00 | 1.00 | 1.00 |

The pH was as follows. Ammonium chloride showed the pH of 4.6 in the saturated amount, ammonium bicarbonate showed the pH of 8.1 in the saturated amount, ammonium carbonate showed the pH of 9.6 in the saturated amount, and ammonium carbamate showed the pH of 9.9 in the saturated amount. In the half saturated amount, the pH was as follows. Ammonium chloride showed the pH of 4.9, ammonium bicarbonate showed the pH of 8.3, ammonium carbonate showed the pH of 9.5, and ammonium carbamate showed the pH of 9.7. On the other hand, in the presence of concentrated aqueous ammonia, ammonium carbonate/concentrated aqueous ammonia (a solution in which ammonium carbonate is saturated in concentrated aqueous ammonia) showed the pH of 11.0 (found value: 10.98). Therefore, it was found that, according to the present invention, a peeling reaction, which is considered as a problem in conventional art, can be suppressed as low as possible by lowering the value of the pH by about 1, thus making it possible to ensure quantitativity.

The results in the case of an ammonium salt were as shown in Fig. 1. In the system using a saturated ammonium carbonate salt and ammonium carbamate, it was shown that a pattern approximated to the reported glycan pattern is obtained, and a glycan is released quantitatively with satisfactory reproducibility. In an ammonium salt other than ammonium carbamate, it was shown that a glycan is not sufficiently released or no action is exerted under the half saturation condition. Therefore, it was shown that a glycan can be efficiently released by treating with the saturated ammonium carbonate and ammonium carbamate.

When examining the results in the case of using an organic amine, it was found that a glycan is released to a given extent. It became apparent that the glycan is not released as in the case of using the saturated ammonium carbonate, and the results of the saturated ammonium carbonate were also shown for comparison. It was shown that the released glycan having a trisaccharide structure is converted into a disaccharide structure as a result of decomposition during the treatment with piperidine, diethylamine or triethylamine. It also became apparent that, although a decomposition product is produced in a small amount, the glycan is slightly released and the condition for release of a glycan using ammonium is better than that in the case of using an organic amine in the treatment with tributylamine or N,N-diethylaniline.

As is apparent from the above description, it was shown that it is effective for quantitative and qualitative analysis to add a saturated amount of an ammonium carbonate powder, preferably an ammonium carbamate powder and to treat a sample solution under the above condition for the purpose of releasing an O-linked glycan of glycoprotein (BSM) used as the sample.

### (Example 2: Confirmation of Qualitativity and Quantitativity of Release of O-Linked Glycan using Bovine Submaxillary Mucin (BSM))

An experiment of confirming that an O-linked glycan can be released using only an ammonium carbonate or ammonium carbamate salt powder and commercially available bovine submaxillary mucin (BSM) similarly to a conventional method (an anhydrous hydrazine decomposition method) by mass spectrometry was performed.

Release of an O-linked glycan from BSM by an anhydrous hydrazine decomposition method was performed as follows.

To 4 mg of BSM, 300 µl of anhydrous hydrazine was added and, after incubation at 60°C for 6 hours, the reaction solution was diluted with H₂O. The diluted reaction solution was applied to a CarboGraph cartridge and the released glycan was acetylated by adsorption, followed by elution with 25% acetonitrile/0.05% trifluoroacetic acid, and thus the released glycan was collected.

Release of an O-linked glycan from BSM using an ammonium carbonate powder or ammonium carbamate powder was performed as follows.

BSM (4 mg) was dissolved in 200 µl of H₂O and 20 µl of the obtained solution was transferred to a 1.5 ml tube, and then 15 mg of an ammonium carbonate powder or 20 mg of an ammonium carbamate powder was added. After stirring by Vortex and collecting at the bottom of the tube by centrifugation, incubation was performed at 60°C for 20 hours. To the reaction solution, 90 µl of 17.4 M acetic acid was added to make the total volume 110 µl. A glycan releasing solution (20 µl) containing 400 µg of BSM was mixed with 2.5 nmol of chitotetraose as an internal standard, and then the mixture was subjected to glycan purification using beads for glycan immobilization.

Immobilization of a BSM-derived O-linked glycan to beads for glycan immobilization and collection of a purified glycan were performed based on an existing method (glycoblotting method using BlotGlyco beads) [Furukawa J-I et al., Anal. Chem., 80, 1094-1101].

### (Mass Spectrometry by MALDI-TOF MS)

The results obtained by performing mass spectrometry after releasing of an O-linked glycan from BSM by an anhydrous hydrazine decomposition method are shown in Fig. 2A, the results obtained by performing mass spectrometry after releasing of an O-linked glycan by an ammonium carbonate powder are shown in Fig. 2B, and the results obtained by performing mass spectrometry after releasing of an O-linked glycan by an ammonium carbamate powder are shown in Fig. 2C.

A sialic acid-containing O-linked glycan was confirmed by mass spectrum in all releasing methods. Peak 5 attributes to an internal standard (chitotetraose). There was not a large qualitative difference between both releasing methods. However, the yield increased entirely and a slight abundance ratio of peaks 6 and 7 was detected particularly in the case of using an ammonium carbamate powder. It was shown that decomposition of the released glycan, which is a non-preferable side reaction, is suppressed as compared with an anhydrous hydrazine decomposition method in the case of using an ammonium carbonate powder and an ammonium carbamate powder. Also, a glycan, which has a large molecular weight and a small abundance ratio, was clearly shown by the technique of the present invention (6 and 7 in Fig. 2C).

As is apparent from the above description, according to the present invention, it was shown that a glycan can be released with satisfactory quantitativity without being inferior in sensitivity or the like as compared with a conventional method, and less decomposition product due to the side reaction is formed. Considering in conjunction with the results of the pH measured in Example 1, it became apparent that a glycan can be released with satisfactory quantitativity without being inferior in sensitivity or the like and also an O-linked glycan can be analyzed without formation of the decomposition product due to the side reaction by adjusting the pH to lower than about 11, more preferably lower than 10.5 to lower than 10, and 8.3 or higher, preferably 9 or higher.

### (Example 3: Example in Case of Using Human Serum)

An experiment of confirming by mass spectrometry that, upon treatment of human serum with saturated ammonium carbamate, glycoprotein glycan can be released from the treated human serum was performed.

Release of an O-linked glycan from serum by an ammonium carbamate powder was performed as follows.

To 20 µl of commercially available human serum, 20 mg of an ammonium carbamate powder was added and, after incubation at 60°C for 40 hours, neutralization was performed by adding 90 µl of glacial acetic acid in ice.

Thereafter, the obtained substance was freeze-dried and dissolved in 20 µl of pure water, and then the released glycan was collected using BlotGlyco beads based on an existing method [Furukawa J-I et al., Anal. Chem., 80, 1094-1101]. The obtained glycan sample was analyzed by mass spectrometry. Amass spectrum is shown in Fig. 3. According to the present invention, it was confirmed that not only an O-linked glycan but also an N-glycan can be released. Also, two kinds of glycans were confirmed as a principal O-linked glycan in human serum.

Considering in conjunction with the results of the pH measured in Example 1, it became apparent that a glycan can be released with satisfactory quantitativity without being inferior in sensitivity or the like and also an O-linked glycan and an N-linked glycan can be analyzed without formation of the decomposition product due to the side reaction by adjusting the pH to lower than about 11, more preferably lower than 10.5 to lower than 10, and 8.3 or higher, preferably 9 or higher.

### (Example 4: Confirmation of Universality of Release of O-Glycan in Biological Sample)

In order to confirm universality of release of an O-glycan in a biological sample by the present invention, release of an O-glycan from a cultured cell extract and a formalin-fixed paraffin-embedded (FFPE) tissue sample and its analysis were performed.

### (Method)

Human breast cancer cell line MCF-7 was used as a cultured cell. Extraction of glycoprotein from the cell was performed in accordance with a conventional method using a surfactant and the obtained protein fraction was treated in accordance with the O-linked glycan releasing method using an ammonium carbamate powder in Example 2.

A rat kidney section (10 µm in thickness) was used as a formalin-fixed paraffin-embedded (FFPE) tissue sample. Four sections were placed in one tube and a protein was extracted using a protein purification kit(Protein Isolation Kit (ToPI-F2), ITSI Biosciences). After precipitation of the protein by methanol-chloroform, the protein was treated in accordance with the O-linked glycan releasing method using an ammonium carbamate powder in Example 2.

The released glycan was purified and collected in accordance with Example 2 and then analyzed by MALDI-TOF mass spectrometry.

### (Results)

The obtained mass spectrum is shown in Fig. 4. In both a cultured cell extract (Fig. 4A) and an FFPE section (Fig. 4B), an O-glycan (asterisk in the figure) in each sample could be identified.

Fig. 4 illustrates analysis of a glycan in a biological sample obtained by a glycan releasing method using ammonium carbamate powder by MALDI-TOF mass spectrometry. A is human breast cancer cell line MCF-7. B is a rat kidney formalin-fixed paraffin-embedded sample. After extraction of a protein fraction, the released glycan was purified and collected by a glycoblotting method using BlotGlyco beads to obtain a mass spectrum. Asterisk represents an O-glycan. Label N in the figure is a peak derived from an N-glycan.

As is apparent from the above description, it was shown that an O-linked glycan can be analyzed by using, in addition to serum, a cultured cell extract, a tissue section and the like as a material.

### (Example 5: Examination of Various Conditions)

In the present example, formation of a glycan when ammonium carbonate is saturated in concentrated aqueous ammonia employed in Patent Document 1 and efficiency of analysis are compared with the present invention.

As described in Example 1, by comparing those obtained by saturated ammonium carbonate in concentrated aqueous ammonia with various basic substances, release of an O-linked glycan from glycoprotein (BSM) is confirmed by mass spectrometry and an effective glycan releasing method is examined.

As described in Example 1, BSM is dissolved in ultrapure water in the concentration of 40 mg/mL and 0.8 mg of the obtained solution is used in each experiment. To BSM, a solution prepared by saturating ammonium carbonate in concentrated aqueous ammonia or various basic substances are added in various concentrations. The reaction conditions are as follows: a treatment time of 20 hours and a treatment temperature of 60°C, and the treatment is performed in duplicate. The pH is measured in the same manner as described in Example 1. The post reaction treatment is also carried out by adjusting the pH within a range from 4 to 5, as described in Example 1, or keeping the pH at neutral (pH about 7). Ultrapure water is added to the obtained solution to make it up to 100 µL and 40 µL of the solution is used in a glycoblotting method using BlotGlyco beads and the released glycan is purified and collected. The collected sample is analyzed by mass spectrometry and release of a glycan is confirmed.

While the present invention has been described by way of the preferred embodiments of the present invention as described above, it is understood that the scope of the present invention should be construed only by the claims. It is understood that the entire contents of patents, patent applications and literatures cited herein should be incorporated by reference herein in the same way as the contents per se are specifically described herein.

### INDUSTRIAL APPLICABILITY

It becomes possible to release a reducing O-linked glycan from biologically relevant molecules in a small amount of a biological sample using the present invention. Since an easy-to-handle ammonia carbonate powder or the like is used, the present invention can be introduced into an automation system toward high throughput analysis, and also can be utilized for analysis of an O-linked glycan linked to a glycoprotein glycan involved in various glycoprotein formulations or drug targets. Also, as the presence of an O-linked glycan having a structure capable of varying with diseases such as cancer is pointed out, an application for quick and simple disease diagnosis can be expected by utilizing the glycan biomarker discovery.

## Claims

1. A method of producing an O-linked glycan from a glycan-binding substance containing the O-linked glycan, the method comprising the steps of:
(A) bringing an ammonium salt or ammonium ion into contact with the glycan-binding substance in the absence of concentrated aqueous ammonia;
(B) neutralizing or acidifying the reaction solution obtained in step (A); and
(C) collecting the released glycan.

2. A method of producing an O-linked glycan from a glycan-binding substance containing the O-linked glycan, the method comprising the steps of:
(A) bringing an ammonium salt or ammonium ion into contact with the glycan-binding substance under the condition in which the pH is about 7 or higher and lower than about 11;
(B) neutralizing or acidifying the reaction solution obtained in step (A); and
(C) collecting the released glycan.

3. A method of detecting an O-linked glycan in a sample, the method comprising the steps of:
(A) bringing an ammonium salt or ammonium ion into contact with the sample in the absence of concentrated aqueous ammonia;
(B) neutralizing or acidifying the reaction solution obtained in step (A); and
(C) analyzing the released glycan.

4. A method of detecting an O-linked glycan in a sample, the method comprising the steps of:
(A) bringing an ammonium salt or ammonium ion into contact with the sample under the condition in which the pH is about 7 or higher and lower than about 11;
(B) neutralizing or acidifying the reaction solution obtained in step (A); and
(C) analyzing the released glycan.

5. A method of detecting an O-linked glycan in a sample, the method comprising the steps of:
(A) adding an ammonium salt to the sample in the absence of concentrated aqueous ammonia;
(B) neutralizing or acidifying the reaction solution obtained in step (A); and
(C) analyzing the released glycan.

6. The method according to any one of claims 1 to 5, wherein the contact with the ammonium salt or ammonium ion, or the addition of the ammonium salt is achieved by adding the ammonium salt in an amount ranging from an amount corresponding to half of the saturated concentration to an amount corresponding to the saturated concentration or more.

7. The method according to any one of claims 1 to 5, wherein the contact with the ammonium salt or ammonium ion, or the addition of the ammonium salt is achieved by the condition in which the ammonium salt is added in an amount corresponding to the saturated concentration or more.

8. The method according to any one of claims 1 to 5, wherein the contact with the ammonium salt or ammonium ion, or the addition of the ammonium salt is achieved by adding a substance capable of generating the ammonium salt or ammonium ion to a solution of the glycan-binding substance in the form of a powder.

9. The method according to any one of claims 1 to 5, wherein the ammonium salt contains at least one salt selected from the group consisting of ammonium carbonate, ammonium bicarbonate and ammonium carbamate.

10. A composition for producing or separating an O-linked glycan from a glycan-binding substance having the O-linked glycan, the composition containing an ammonium salt or ammonium ion.

11. The composition according to claim 10, wherein the ammonium salt or ammonium ion is ammonium bicarbonate or ammonium carbamate.

12. An ammonium salt or ammonium ion for producing or separating an O-linked glycan from a glycan-binding substance having the O-linked glycan.
